# EUROPÄISCHE PATENTSCHRIFT

(11) **EP 2 944 312 B1**
(45) Veröffentlichungstag und Bekanntmachung des Hinweises auf die Patenterteilung: **26.01.2022**
(21) Anmeldenummer: 14168626.1
(22) Anmeldetag: 16.05.2014
(51) Int. Cl.: A61K 31/7008, A61K 31/7084, A61K 31/737, A61K 35/32, A61K 45/06, A61P 19/02

(54) **KOMBINATIONSPRÄPARAT, UMFASSEND GLUCOSAMIN UND CHONDROITINSULFAT**
COMBINATION PREPARATION, COMPRISING GLUCOSAMINE AND CHONDROITIN SULPHATE
PRÉPARATION DE COMBINAISON, COMPRENANT DE LA GLUCOSAMINE ET DU SULFATE DE CHONDROÏTINE

(43) Veröffentlichungstag der Anmeldung: 18.11.2015
(73) Patentinhaber: TP Tumapharma Ltd, 3174 Sliema (MT)
(72) Erfinder: Huber, Johannes, 1130 Wien (AT)
(74) Vertreter: SONN Patentanwälte OG

(56) Entgegenhaltungen:
- WO-A1-2009/153200
- HATHCOCK ET AL: "Risk assessment for glucosamine and chondroitin sulfate", REGULATORY TOXICOLOGY AND PHARMACOLOGY, ACADEMIC PRESS,NEW YORK, NY, US, Bd. 47, Nr. 1, 21. November 2006 (2006-11-21), Seiten 78-83, XP005727134, ISSN: 0273-2300, DOI: 10.1016/J.YRTPH.2006.07.004
- SHINO NEMOTO ET AL: "Das Wunder der über 120-Jährigen", SPEKTRUM DER WISSENSCHAFT, November 2004 (2004-11), Seiten 70-75, XP055148113,

## Beschreibung

Die Erfindung betrifft pharmazeutische Kombinationspräparate, gemäß den beigefügten Ansprüchen umfassend Glucosaminsulfat und Chondroitinsulfat, insbesondere zur Vorbeugung und Behandlung von Osteoarthritis (OA).

OA ist ein aus dem Englischen abgeleiteter Fachbegriff und bezeichnet eine degenerative Gelenkserkrankung, sie Arthrose. Im allgemeinen versteht man unter Arthrose einen "Gelenkverschleiß", der das altersübliche Maß übersteigt. Ursächlich werden ein Übermaß an Belastung (etwa erhöhtes Körpergewicht), angeborene oder traumatisch bedingte Ursachen, wie Fehlstellungen der Gelenke, oder auch knöcherner Deformierung durch Knochenerkrankungen wie Osteoporose gesehen. Die Arthrose kann ebenfalls als Folge einer anderen Erkrankung, beispielsweise einer Gelenkentzündung (Arthritis) entstehen (sekundäre Arthrose) oder mit überlastungsbedingter Ergussbildung (sekundäre Entzündungsreaktion) einhergehen (aktivierte Arthrose). Dabei ist allerdings die OA streng zu unterscheiden von der Rheumatoiden Arthritis (engl. Rheumatoid Arthritis, RA), einer chronisch entzündlichen Gelenkerkrankung.

Grundsätzlich können alle Gelenke von arthrotischen Veränderungen betroffen werden, häufig ist die Erkrankung am häufigsten im Kniegelenk lokalisiert. Arthrose ist weltweit die häufigste Gelenkerkrankung.

Die Therapie der Arthrose verfolgt grundsätzlich zwei Ziele: Schmerzfreiheit unter üblicher Belastung und Verhinderung mechanischer Einschränkungen oder Veränderungen eines Gelenkes.

Neben der Verwendung von Schmerzmitteln (Diclofenac, Ibuprofen oder Naproxen; Etoricoxib und Celecoxib; in schweren Fällen: stark wirksame Analgetika vom Morphin-Typ) kommen dabei vor allem operative Verfahren zum Einsatz, z.B. Beseitigung mechanischer Risikofaktoren wie Gelenkdysplasien oder anderer Ursachen, die zu vermehrter Druckbelastung des Gelenkknorpels führen, Regeneration oder Transplantation des Gelenkknorpels oder endoprothetischer Ersatz der Gelenkfläche oder des gesamten Gelenks.

Auch die Substanzen Glucosaminsulfat (GS) und Chondroitinsulfat (CS) wurden zur Behandlung von Arthrose vorgeschlagen (Cohen et al., J. Rheumatol. 30 (2003), 523-528; Simanek et al., Biomed. Papers 149 (2005), 51-56). Beide Substanzen sind toxikologisch unbedenklich und weisen vernachlässigbare Nebenwirkungen auf. Sie werden sowohl zur Schmerzlinderung als auch zur Verbesserung der Gelenksbeweglichkeit verwendet sind; auch eine entzündungshemmende Wirkung wird ihnen zugeschrieben.

Es besteht jedoch weiterhin ein großer Bedarf an Mitteln zur Vorbeugung und Behandlung von OA, insbesondere an Mitteln, die den Ausbruch oder den Verlauf der Erkrankung verzögern können, und trotzdem ohne wesentliche Nebenwirkungen langfristig eingenommen werden können.

Daher betrifft die vorliegende Erfindung eine Zusammensetzung, umfassend Glucosamin (als Glucosaminhydrochlorid oder Glucosaminsulfat; gemeinsam als GA bezeichnet), Chondroitinsulfat (CS) und Nicotinamidadenindinukleotid in oxidierter Form (NAD).

Das erfindungsgemäße Kombinationspräparat ist insbesondere für die Behandlung einer altersbedingten Arthrose vorteilhaft. Die bevorzugte Patienten-Zielgruppe der vorliegenden Erfindung, die OA-Patienten oder Patienten, für die ein Risiko besteht, dass sie OA entwickeln, umfassen Personen, die unter den genannten Symptomen leiden - aber nicht auf Grund extremer sportlicher Betätigung eine Arthrose entwickelten. Um einer (altersbedingten) Arthrose gegensteuern zu können, ist eine Stärkung der Skelett-Muskulatur unumgänglich. Diese ist allerdings v.a. altersbedingt (v.a. aber in Abhängigkeit der Beanspruchung) funktionell beeinträchtigt und nicht mehr in der Lage, die notwendige Menge an nuklearem NAD zu bilden um den notwendigen physiologischen zellulären Mindestgehalt zu gewährleisten. Damit steht aber nicht die mitochondriale ATP-Energiegewinnung über NADH/OXPHOS im Vordergrund, sondern mehr das "anti-Ageing"-Potential von NAD (s. zB. Nemoto et al., SdW 2004, 70-75). Die erfindungsgemäße Kombination wirkt daher auf mehreren Ebenen der OA entgegen und ergänzt die jeweils andere Behandlungsstrategie. Die erfindungsgemäße Zusammensetzung eignet sich daher besonders gut für die Behandlung der altersbedingten OA.

CS ist ein Glykosaminoglykan (GAG). GAGs sind linear aus sich wiederholenden Disacchariden aufgebaute, saure Polysaccharide. CS besteht aus β-(1→4)-glykosidisch verknüpften Glukur-onyl-β-(1→3)-N-Acetylgalaktosamin-Disacchariden, wobei D-Glukuronsäure teilweise in L-Iduronsäure umgewandelt wird. Wenn mehr als 10 % des Uronats als Iduronat vorliegen, spricht man von Dermatansulfat. Der Grad der Sulfatierung von Chondroitinsulfat und Dermatansulfat beträgt etwa ein Sulfatrest pro Disaccharid. Das Sulfat in CS liegt als 4-Sulfat oder 6-Sulfat vor. Beim Dermatansulfat kann aufgrund Epimerisierung an C5 auch Glucuronat vorkommen. Die Molare Masse liegt zwischen 10 und 50 kDa. CS kommt im Bindegewebe (besonders im Knorpel und in Synovia). CS wird aus Knorpelgewebe von Schweinen, Kühen Haifischen und Vögeln gewonnen. Knorpelextrakte von Kälbern und auch von Haien (Kälberknorpel; Haifischknorpel) enthalten 10-30% CS (Gonzalez et al., Biol. Pharm. Bull. 24 (2001), 1097-1101).

GA ist der allgemein anerkannte Trivialname für 2-Amino-2-desoxy-α/β-D-glucopyranose. Es ist also ein Derivat der D-Glucose, von der es sich nur durch die Substitution der Hydroxygruppe am zweiten Kohlenstoff durch eine Aminogruppe unterscheidet. Industriell wird Glucosamin aus Chitin hergestellt. Dazu ist eine Deacetylierung erforderlich, sowie eine hydrolytische Spaltung des Polymers in die Monomere. Beide Schritte verlaufen gleichzeitig in heißer Salzsäure. Der Ausgangsstoff Chitin wird überwiegend als Sekundärrohstoff aus den Abfällen der Fischerei von Krustentieren (Krabben, Garnelen) gewonnen. Prinzipiell kann das Glucosamin auch aus dem Chitin von Insekten (z. B. Seidenspinnerraupen oder Bienen) oder aus Pilzen (z. B. Aspergillus niger) gewonnen werden; es kann auch synthetisch hergestellt werden.

CS und GA sind auch in Simanek et al., 2005 näher beschrieben.

2006 wurde vom NIH USA eine Studie zur Wirksamkeit von CS und/oder GA bei Arthrose des Knies initiiert. Diese doppelblinde, randomisierte, placebokontrollierte Studie an 1.585 Patienten legte für die Kombination von CS und GA eine mögliche Wirksamkeit bei mittleren und schweren Knieschmerzen nahe (Clegg et al., 2006).

Eine doppelblinde, randomisierte, placebokontrollierte Studie von 2013, durchgeführt von der Universität Sydney mit 605 Probanden (Alter 45-75 Jahre, Kniegelenksarthrose) konnte eine statistisch signifikante Abschwächung des Gelenkspaltrückgangs beobachten. Es wurde dabei über 2 Jahre täglich 2 x 750 mg Glucosaminsulfat und 2 x 400 mg Chondroitinsulfat eingenommen. Ein Rückgang der Gelenkschmerzen konnte bei allen Gruppen [1) Glucosaminsulfat 2 x 750 mg, 2) Chondroitinsulfat 2 x 400 mg, 3) Glucosaminsulfat 2 x 750 mg + Chondroitinsulfat 2 x 400 mg, 4) Placebo] - ohne signifikante Unterschiede zwischen den Gruppen - beobachtet werden (Fransen et al., Ann Rheum Dis. (2014), doi: 10.1136/annrheumdis-2013-203954).

WO 2009/153200 A1 betrifft Zubereitungen enthaltend Glucosamin und/oder Chondroitin zur Prophylaxe und Therapie der Arthrose als auch zur Unterstützung des Immunsystems. Hathcock et al. Regulatory Toxicology and Pharmacology 47.1 (2007): 78-83) betrifft die Sicherheit der Einnahme von Glucosamin und Chondroitinsulfat.

Gemäß der vorliegenden Offenbarung werden die an sich bekannten Zusammensetzungen aus CS und GA durch den Zusatz von Nikotinamid-Dinukleotid-Verbindungen, NAD, verbessert. Im offenbarten Präparat NAD bedeutet die oxidierte Form (NAD⁺, NADP⁺; werden hierin der Einfachheit halber mit "NAD" und "NADP" bezeichnet) der bekannten Empfindlichkeit der reduzierten Form ist das Vorsehen der oxidierten Form in der vorliegenden Erfindung offenbart.

NAD ist ein Coenzym, das formal ein Hydridion überträgt (Zwei-Elektronen/Ein-Proton). Es ist an zahlreichen Redoxreaktionen des Stoffwechsels der Zelle beteiligt. NADPH ist ein Derivat des NADH, nämlich die (am Riboseteil, C2') phosphorylierte Form des Coenzyms NADH. Zwischen NADH und NADPH gibt es in den meisten biochemischen Reaktionen einen fundamentalen Unterschied: NADH wird im Katabolismus aus Glykolyse und Citratzyklus gewonnen und in der Atmungskette oxidiert, um ATP zu erzeugen. Dagegen fungiert NADPH im Anabolismus als Reduktionsmittel, es dient in der reduzierenden Biosynthese als Lieferant von Elektronen und Protonen. Im Rahmen der vorliegenden Erfindung wird NAD eingesetzt.

Dabei können die Bestandteile des erfindungsgemäßen Kombinationspräparates vorzugsweise in den Konzentrationen oder Mengen zur Anwendung kommen, in denen ihre Wirksamkeit bereits in klinischen Studien belegt wurden (zB. Fransen et al., 2014; Morelli et al., Am. Fam. Phys. 67 (2003), 339-344; Widli et al., Ann. Rheum. Dis. 70 (2011), 982-989); es ist aber auch möglich, höhere Dosierungen einzusetzen, insbesondere, da die erfindungsgemäßen Komponenten des Kombinationspräparates von ihrer Toxikologie und ihrem Nebenwirkungsspektrum völlig unbedenklich sind. Auch niedrigere Dosen sind anwendbar, wenn die Verabreichungsfrequenz und/oder die Verabreichungsdauer entsprechend erhöht wird. Bevorzugte Mengen in einem als Einzeldosis zur Verfügung gestellten erfindungsgemäßen Kombinationspräparat sind daher, unabhängig voneinander, 0,01 bis 10,0 g, vorzugsweise 0,1 bis 5,0 g, insbesondere 0,3 bis 2,0 g, GA und/oder 0,01 bis 10,0 g, vorzugsweise 0,1 bis 5,0 g, insbesondere 0,3 bis 2,0 g, CS und/oder 0,001 bis 1 g, vorzugsweise 0,01 bis 0,5 g, insbesondere 0,05 bis 0,3 g, NAD.

Da das erfindungsgemäße Kombinationspräparat bevorzugt zur Behandlung der OA eingesetzt wird (und die Behandlung der OA oftmals die Behandlung mit Schmerzmitteln einschließt), kann es weiters ein Schmerzmittel, insbesondere Celecoxib, Etoricoxib, Acetaminophen, Diclofenac, Ibuprofen und/oder Naproxen enthalten.

Bevorzugte Verabreichungswege für das erfindungsgemäße Kombinationspräparat ist die orale oder topische Verabreichung (obgleich andere Verabreichungswege, insbesondere die Verabreichung direkt ins Gelenk (etwa im Zuge einer athroskopischen Behandlung oder per Injektion) natürlich auch möglich ist). Vorzugsweise ist aber die Zusammensetzung zur oralen oder topischen Verabreichung hergerichtet. Hierbei sind die für derartige Verabreichungen vorgesehenen etablierten Formulierungen auch für die vorliegende Zusammensetzung anwendbar, insbesondere diejenigen Verabreichungsformulierungen, die bereits für CS und GA enthaltende Präparate beschrieben wurden. Für die zusätzliche Anwesenheit von NAD und/oder NADP sind in der Regel keine zusätzlichen Erfordernisse bei der Formulierung gegeben; wenn NADH und/oder NADPH zum Einsatz kommen, kann eine Stabilisierung wie z.B. in US 4,970,200 A oder EP 0 697 859 A offenbart, empfehlen.

Demgemäß wird die oral zu verabreichende Zusammensetzung vorzugsweise in Form einer Tablette oder Kapsel zur Verfügung gestellt, insbesondere mit einer magensaftresistenten Schutzhülle. Gegebenenfalls können die Inhaltsstoffe (jeweils als Einzelstoff und/oder als Kombinationspräparat in granulierter Form vorgelegt werden.

Das zur topischen Verabreichung hergerichtete Kombinationspräparat wird vorzugsweise in Form einer Salbe oder eines Gels zur Verfügung gestellt.

Das erfindungsgemäße Kombinationspräparat kann darüber hinaus weitere Inhaltsstoffe aufweisen, etwa geeignete pharmazeutisch akzeptable Trägerstoffe, Exzipienten oder Verdünnungsmittel. Besonders bevorzugte weitere Inhaltsstoffe sind dabei Puffersubstanzen, Stabilisatoren und Konservierungsmittel. Weiters ist besonders der Zusatz hilfreicher Ionen bevorzugt. Daher ist eine besonders bevorzugte Ausführungsform der erfindungsgemäßen Zusammensetzung dadurch gekennzeichnet, dass sie weiters Calcium-, Natrium-, Magnesium-, Kalium-, Zink-, Eisen-, und/oder Phosphat-Ionen umfasst.

Gemäß einer bevorzugten Ausführungsform enthält das erfindungsgemäße Kombinationspräparat CS in Form von Kälber- oder Haifischknorpelpulver, insbesondere in Form von Haifischknorpelpulver (Pearson et al., Mol. Nutr. Food Res. 51 (2007), 1020-1030).

Gemäß einem weiteren Aspekt betrifft die vorliegende Erfindung ein Kombinationspräparat, wie hierin beschrieben, zur Anwendung in einem therapeutischen Behandlungsverfahren, insbesondere zur Anwendung zur Vorbeugung oder Behandlung von OA (Arthrose). Das erfindungsgemäße Kombinationspräparat wird dabei natürlich als pharmazeutisches Kombinationspräparat vorgesehen, also hergestellt nach "Good Manufacturing Practice" (GMP) und mit allen weiteren für eine arzneimittelrechtliche Vermarktung erforderlichen Eigenschaften, insbesondere mit (in) einem pharmazeutisch akzeptablen Träger.

Dabei wird das erfindungsgemäße Kombinationspräparat an einen Patienten mit diagnostizierter OA oder mit einem Risiko, eine OA zu entwickeln, in einer effizienten Dosierung verabreicht. Wie erwähnt, kann das erfindungsgemäße Kombinationspräparat über längere Zeit bedenkenlos eingenommen bzw. appliziert werden, so dass Anwendungszeiträume über mehrere Monate oder Jahre problemlos sind. Vorzugsweise wird daher das erfindungsgemäße Kombinationspräparat täglich ein bis dreimal eingenommen bzw. angewendet. Der bevorzugte Behandlungszeitraum ist dabei zumindest 3 Monate, vorzugsweise zumindest 6 Monate, insbesondere mindestens 1 Jahr. Geeignete Behandlungszeiträume sind aber weiters mindestens 2 Jahre, mindestens 5 Jahre, mindestens 10 Jahre, mindestens 20 Jahre oder sogar mindestens 30 Jahre.

Das erfindungsgemäße Kombinationspräparat ist vor allem zur Behandlung der altersbedingten Schwächung von Knochen und Skelettmuskulatur vorgesehen, insbesondere zur Langzeittherapie und -prophylaxe dieser Erkrankung.

Das erfindungsgemäße Kombinationspräparat kann aber auch nicht-therapeutisch verwendet werden, indem es bevorzugt als Nahrungsergänzungsmittel eingesetzt wird.

Die therapeutische Behandlung von OA schließt in der Regel eine Schmerzbehandlung mit ein. Demgemäß kann auch das erfindungsgemäße Kombinationspräparat ein Schmerzmittel unmittelbar enthalten oder in Form eines Sets in Kombination mit einem Schmerzmittel angeboten werden. Dabei kann das Set ein erfindungsgemäßes Kombinationspräparat und ein Schmerzmittel umfassen, oder aber ein Kombinationspräparat, enthaltend GA und CS, ein Präparat, enthaltend NAD, sowie gegebenenfalls ein Schmerzmittel.

Die Erfindung wird anhand der nachfolgenden Beispiele näher erläutert, ohne aber darauf eingeschränkt zu sein.

### Beispiele:

### 1. Herstellung des erfindungsgemäßen Kombinationspräparats (orale Verabreichungsform)

Die folgenden Zusammensetzungen werden in Form von Kapseln mit magensaftresistenter Hülle (jeweils mit Calcium-, Natrium-, Magnesium-, Kalium-, Zink-, Eisen-, und Phosphat-Ionen) vorgesehen:
Präparat 1
   1500 mg GA
   1500 mg CS
   100 mg NAD
Präparat 2
   1500 mg GA
   1500 mg CS
   200 mg NAD
Präparat 3
   1000 mg GA
   500 mg CS
   100 mg NAD
Präparat 4
   750 mg GA
   400 mg CS
   100 mg NAD
Präparat 5
   100 mg GA
   100 mg CS
   100 mg NAD
Präparat 6 (nicht erfindungsgemäß)
   100 mg GA
   100 mg CS
   100 mg NADP
Präparat 7 (nicht erfindungsgemäß)
   750 mg GA
   400 mg CS
   100 mg NADP
Präparat 8
   2000 mg GA
   2000 mg CS
   500 mg NAD
Präparat 9
   3000 mg GA
   1000 mg CS
   400 mg NAD
Präparat 10
   2000 mg GA
   1000 mg CS
   300 mg NAD

### 2. Anwendungsbeobachtung

Die Präparate gemäß der vorliegenden Erfindung werden an Patientinnen kontrolliert verabreicht. Die Patientinnen werden osteodensitometrisch überwacht; auch eine Bestimmung der Muskelleistungsparameter wird in regelmäßigen Abständen durchgeführt.

## Patentansprüche

1. Kombinationspräparat, umfassend Glucosamin (als Glucosaminhydrochlorid oder Glucosaminsulfat; GA), Chondroitinsulfat (CS) und Nicotinamidadenindinukleotid in oxidierter Form (NAD).

2. Kombinationspräparat nach Anspruch 1, **dadurch gekennzeichnet, dass** es, unabhängig voneinander, 0,01 bis 10,0 g, vorzugsweise 0,1 bis 5,0 g, insbesondere 0,3 bis 2,0 g, GA und 0,01 bis 10,0 g, vorzugsweise 0,1 bis 5,0 g, insbesondere 0,3 bis 2,0 g, CS und 0,001 bis 1 g, vorzugsweise 0,01 bis 0,5 g, insbesondere 0,05 bis 0,3 g, NAD enthält.

3. Kombinationspräparat nach Anspruch 1 oder 2, **dadurch gekennzeichnet, dass** es ein Schmerzmittel, insbesondere Celecoxib, E-toricoxib, Acetaminophen, Diclofenac, Ibuprofen und/oder Naproxen enthält.

4. Kombinationspräparat nach einem der Ansprüche 1 bis 3, **dadurch gekennzeichnet, dass** es zur oralen Verabreichung hergerichtet ist.

5. Kombinationspräparat nach Anspruch 4, **dadurch gekennzeichnet, dass** es in Form einer Tablette oder Kapsel vorliegt und eine magensaftresistente Schutzhülle aufweist.

6. Kombinationspräparat nach Anspruch 4 oder 5, **dadurch gekennzeichnet, dass** es in granulierter Form vorliegt.

7. Kombinationspräparat nach einem der Ansprüche 1 bis 3, **dadurch gekennzeichnet, dass** es zur topischen Verabreichung hergerichtet ist.

8. Kombinationspräparat nach Anspruch 7, **dadurch gekennzeichnet, dass** es in Form einer Salbe oder eines Gels vorliegt.

9. Kombinationspräparat nach einem der Ansprüche 1 bis 8, **dadurch gekennzeichnet, dass** es Calcium-, Natrium-, Magnesium-, Kalium-, Zink-, Eisen-, und/oder Phosphat-Ionen umfasst.

10. Kombinationspräparat nach einem der Ansprüche 1 bis 9, **dadurch gekennzeichnet, dass** es CS in Form von Kälber- oder Haifischknorpelpulver, insbesondere in Form von Haifischknorpelpulver, enthält.

11. Kombinationspräparat nach einem der Ansprüche 1 bis 10, zur Anwendung in einem therapeutischen Behandlungsverfahren, insbesondere als pharmazeutisches Kombinationspräparat zur Anwendung zur Vorbeugung oder Behandlung von Osteoarthritis (OA).

12. Kombinationspräparat zur Anwendung nach Anspruch 11, zur Behandlung der altersbedingten Schwächung von Knochen und Skelettmuskulatur.

13. Set, umfassend ein Kombinationspräparat nach einem der Ansprüche 1 bis 10 und ein Schmerzmittel; oder ein Kombinationspräparat, enthaltend GA und CS, ein Präparat, enthaltend NAD (in oxidierter Form), sowie gegebenenfalls ein Schmerzmittel.

## Claims

1. Combination preparation comprising glucosamine (as glucosamine hydrochloride or glucosamine sulphate; GA), chondroitin sulphate (CS) and nicotinamide adenine dinucleotide in oxidised form (NAD).

2. Combination preparation according to claim 1, **characterised in that**, independently of one another, it contains 0.01 to 10.0 g, preferably 0.1 to 5.0 g, in particular 0.3 to 2.0 g, GA and 0.01 to 10.0 g, preferably 0.1 to 5.0 g, in particular 0.3 to 2.0 g, CS and 0.001 to 1 g, preferably 0.01 to 0.5 g, in particular 0.05 to 0.3 g, NAD.

3. Combination preparation according to either claim 1 or claim 2, **characterised in that** it contains a pain reliever, in particular celecoxib, e-toricoxib, acetaminophen, diclofenac, ibuprofen and/or naproxen.

4. Combination preparation according to any of claims 1 to 3, **characterised in that** it is prepared for oral administration.

5. Combination preparation according to claim 4, **characterised in that** it is in the form of a tablet or capsule and has an enteric-coated protective cover.

6. Combination preparation according to either claim 4 or claim 5, **characterised in that** it is in granulated form.

7. Combination preparation according to any of claims 1 to 3, **characterised in that** it is prepared for topical administration.

8. Combination preparation according to claim 7, **characterised in that** it is in the form of an ointment or a gel.

9. Combination preparation according to any of claims 1 to 8, **characterised in that** it comprises calcium, sodium, magnesium, potassium, zinc, iron and/or phosphate ions.

10. Combination preparation according to any of claims 1 to 9, **characterised in that** it contains CS in the form of calf or shark cartilage powder, in particular in the form of shark cartilage powder.

11. Combination preparation according to any of claims 1 to 10 for use in a therapeutic treatment method, in particular as a pharmaceutical combination preparation for use in the prevention or treatment of osteoarthritis (OA).

12. Combination preparation for use according to claim 11 for the treatment of age-related weakening of bones and skeletal muscles.

13. Set comprising a combination preparation according to any of claims 1 to 10 and a pain reliever;
or a combination preparation containing GA and CS, a preparation containing NAD (in oxidised form), and optionally a pain reliever.

## Revendications

1. Préparation combinée comprenant de la glucosamine (sous forme de chlorhydrate de glucosamine ou de sulfate de glucosamine ; GA), du sulfate de chondroïtine (CS) et du nicotinamide adénine dinucléotide sous forme oxydée (NAD).

2. Préparation combinée selon la revendication 1, **caractérisée en ce qu'**elle contient, indépendamment les uns des autres, 0,01 à 10,0g, de préférence 0,1 à 5,0g, en particulier 0,3 à 2,0 g de GA, 0,01 à 10,0 g, de préférence 0,1 à 5,0 g, en particulier 0,3 à 2,0 g de CS et 0,001 à 1 g, de préférence 0,01 à 0,5 g, en particulier 0,05 à 0,3 g de NAD.

3. Préparation combinée selon la revendication 1 ou 2, **caractérisée en ce qu'**elle contient un analgésique, en particulier le célécoxib, l'étoricoxib, l'acétaminophène, le diclofénac, l'ibuprofène et/ou le naproxène.

4. Préparation combinée selon l'une des revendications 1 à 3, **caractérisée en ce qu'**elle est destinée à être administrée par voie orale.

5. Préparation combinée selon la revendication 4, **caractérisée en ce qu'**elle se présente sous la forme d'un comprimé ou d'une capsule et comporte une enveloppe protectrice gastro-résistante.

6. Préparation combinée selon la revendication 4 ou 5, **caractérisée en ce qu'**elle se présente sous forme de granulés.

7. Préparation combinée selon l'une des revendications 1 à 3, **caractérisée en ce qu'**elle est destinée à être administrée par voie topique.

8. Préparation combinée selon la revendication 7, **caractérisée en ce qu'**elle se présente sous la forme d'une pommade ou d'un gel.

9. Préparation combinée selon l'une des revendications 1 à 8, **caractérisée en ce qu'**elle comprend des ions calcium, sodium, magnésium, potassium, zinc, fer, et/ou phosphate.

10. Préparation combinée selon l'une des revendications 1 à 9, **caractérisée en ce qu'**elle contient du CS sous forme de poudre de cartilage de veau ou de requin, en particulier sous forme de poudre de cartilage de requin.

11. Préparation combinée selon l'une des revendications 1 à 10, destinée à être utilisée dans un procédé de traitement thérapeutique, en particulier en tant que préparation pharmaceutique combinée destinée à être utilisée pour la prévention ou le traitement de l'ostéoarthrite (OA).

12. Préparation combinée destinée à être utilisée selon la revendication 11, pour le traitement de l'affaiblissement des os et des muscles squelettiques dû à l'âge.

13. Kit comprenant une préparation combinée selon l'une des revendications 1 à 10 et un analgésique ; ou une préparation combinée contenant de la GA et du CS, une préparation contenant du NAD (sous forme oxydée), et éventuellement un analgésique.
